# EUROPÄISCHE PATENTSCHRIFT

(11) **EP 2 887 901 B1**
(45) Veröffentlichungstag und Bekanntmachung des Hinweises auf die Patenterteilung: **02.09.2020**
(21) Anmeldenummer: 13737803.0
(22) Anmeldetag: 17.07.2013
(51) Int. Cl.: A61B 18/12

(54) **HOCHFREQUENZCHIRURGISCHE VORRICHTUNG**
HIGH-FREQUENCY SURGICAL DEVICE
DISPOSITIF CHIRURGICAL À HAUTE FRÉQUENCE

(30) Priorität: 22.08.2012 DE 102012016563
(43) Veröffentlichungstag der Anmeldung: 01.07.2015
(73) Patentinhaber: Olympus Winter & Ibe GmbH, 22045 Hamburg (DE)
(72) Erfinder: JÜRGENS, Thorsten, 20359 Hamburg (DE); TREBBELS, Dennis, 23611 Bad Schwartau (DE); FREITAG, Thomas, 22089 Hamburg (DE); BARTOLIC, Josef, 76135 Karlsruhe (DE)
(74) Vertreter: Hausfeld, Norbert
(86) Internationale Anmeldenummer: PCT/EP2013/002118
(87) Internationale Veröffentlichungsnummer: WO 2014/029455

(56) Entgegenhaltungen:
- EP-A2- 1 637 087
- WO-A1-00/54683
- US-A1- 2005 149 012

## Beschreibung

Die Erfindung betrifft eine hochfrequenzchirurgische Vorrichtung nach dem Oberbegriff des Anspruches 1.

Aus EP 1 637 087 A2 ist ein gattungsgemäßes elektrochirurgisches Instrument mit einer oder mehrerer an einem distalen Ende eines Schaftes angeordneten Elektroden bekannt, wobei das Instrument an eine Hochfrequenzquelle anschließbar ist, um im Bereich der Elektroden in einer elektrisch leitenden Flüssigkeit ein ionisiertes Flüssigkeitsplasma zu erzeugen.

US 2005/0149012 A1 beschreibt ein Verfahren zur Hautbehandlung mit einem Plasma, wobei unter Bereitstellung eines Hochfrequenzstromes aus einer Hochfrequenzquelle an zumindest einer Elektrode eines Behandlungsinstrumentes ein elektrisches Feld erzeugt wird, das ein an die Elektrode hingeleitetes Gas elektrisch zündet, um das Plasma im Bereich der Elektroden zu bilden.

WO 00/54683 A1 beschreibt ein Schaftinstrument zur Plasmabehandlung von Zellgewebe im Pulsbetrieb, bei dem eine am distalen Endbereich des Schaftes angeordnete Aktivelektrode mit einem Aktivpol einer Hochfrequenzquelle verbindbar ist und eine Neutralelektrode, die mit elektrischer Masse verbunden ist. Es ist eine Schalteinrichtung vorgesehen, die die Aktivelektrode während eines elektrischen Pulses mit dem Aktivpol der Hochfrequenzquelle und in den Pulpausen mit der elektrischen Masse verbindet.

Eine weitere gattungsgemäße Vorrichtung ist aus der DE 10 2007 054 438 A1 bekannt. Diese und andere bekannte gattungsgemäße Konstruktionen werden mit der Elektrode in Flächenkontakt auf eine Gewebeoberfläche gebracht. Bei eingeschaltetem Hochfrequenzstrom wird die Gewebeoberfläche stark erhitzt. Je nach Anwendungszweck kann dies zum Kauterisieren, also zum Verschließen blutender Oberflächen benutzt werden, oder zum Vaporisieren, also zur Verdampfung und somit Abtragung von Gewebevolumen.

Wesentliche, aber nicht die einzigen Anwendungsgebiete sind die Urologie und die Gynäkologie. Dort wird in Flüssigkeit gearbeitet. Monopolare Anordnungen verwenden schlecht leitende Flüssigkeit und Stromfluss vom Gewebekontakt durch den Körper bis zu einer außen am Körper angeordneten Neutralelektrode. Neuere Methoden arbeiten in gut leitender Flüssigkeit und mit einer Neutralelektrode, die mit der Flüssigkeit in Kontakt steht und den wesentlichen Stromfluss durch diese hindurch erzeugt.

Neuerdings wird sehr häufig in gut leitender Flüssigkeit, z.B. in Saline (Kochsalzlösung) vaporisiert. Dabei wird mit einer auf der Elektrode entstehenden Plasmaschicht die Gewebeoberfläche sehr stark erhitzt und verdampft. Damit können größere Gewebevolumina sehr leicht abgetragen werden, z. B. bei der Behandlung der Prostata.

Mit der Elektrode können besonders gut flächige Arbeiten an Gewebeoberflächen durchgeführt werden. Vorteilhaft bei der bekannten Konstruktion ist die große Elektrodenfläche, die eine hohe Arbeitsleistung ermöglicht. Nachteilig ist dabei allerdings das schlechte Zündverhalten.

Das Zündverhalten ist von der Stromdichte auf der Elektrodenoberfläche abhängig. Dabei hängt die Stromdichte von der Grösse der Elektrodenoberfläche ab. Eine vorteilhaft große Elektrodenfläche führt zu geringer Stromdichte und zu schlechtem Zünden. Die Zündung kommt in vielen Fällen nur zögernd und unsicher zustande. Schon bei leichter Verschlechterung des Gewebekontaktes bricht das Plasma zusammen und muss erneut gezündet werden. Der Operateur muss daher sehr feinfühlig vorgehen.

Hinzu kommt ein Wärmeproblem. Wenn die Vorrichtung häufig zünden muss, kann die Gewebetemperatur in unerwünschter Weise ansteigen.

Die Aufgabe der vorliegenden Erfindung besteht darin, bei einer gattungsgemäßen Vorrichtung die Handhabung und das Wärmeproblem zu verbessern.

Diese Aufgabe wird mit den Merkmalen des Kennzeichnungsteiles des Anspruches 1 gelöst.

Erfindungsgemäß werden mehrere Elektroden betrieben, und zwar zunächst eine erste und dann ein zweite oder mehrere weitere zusätzlich. Alle Elektroden sind mit demselben Pol des Hochfrequenzgenerators verbunden. Das bedeutet, dass die Elektroden zunächst nur mit einem Flächenteil arbeitet und es werden dann weitere Flächenteile zugeschaltet. Das bedeutet aber, dass zu Beginn nur eine kleine Elektrodenfläche arbeitet. Das begünstigt eine hohe Stromdichte, also sicheres Zünden. Wenn das Plasma erst einmal brennt, kann die zweite Elektrode zugeschaltet werden und sofort zünden. Mit der Erfindung lassen sich also die Vorteile des guten Zündverhaltens einer kleinen Elektrode mit den Vorteilen der hohen Arbeitsleistung einer großen Elektrode kombinieren.

Weitere vorteilhafte Merkmale des Anspruches 1 sorgen dafür, dass die Steuereinrichtung automatisch die Zuschaltung vornimmt. Der Operateur muss sich nicht mehr darum kümmern. Er verfügt stets über ein sicheres Zündverhalten.

Vorteilhaft gemäß Anspruch 2 wird das Zuschalten vom Gewebekontakt abhängig gemacht. Das kann mit anderen Maßnahmen kombiniert werden und ergibt wiederum einen verbesserten Arbeitskomfort für den Operateur.

Dabei kann der Gewebekontakt mit den Merkmalen des Anspruches 2 automatisch erkannt werden. Zur Erkennung werden vorteilhaft zwei ohnehin vorhandene Elektroden verwendet, zwischen denen eine Impedanzmessung erfolgt.

Vorteilhaft gemäß Anspruch 3 kann die Zuschaltung auch von Hand betätigt werden, bzw. mit dem üblichen Fußschalter.

Vorteilhaft sind die Merkmale des Anspruches 4 vorgesehen. Die Unterteilung des zweiten Elektrode in mehrere Teile kann Vorteile bei der Anordnung der Elektrode auf beliebig geformter Arbeitsfläche ermöglichen.

Anspruch 5 stellt die Elektrodeneinrichtung mit der Steuereinrichtung der erfindungsgemäßen Vorrichtung unter Schutz.

Anspruch 6 stellt den Hochfrequenzgenerator mit der Steuereinrichtung der erfindungsgemäßen Vorrichtung unter Schutz.

In der Zeichnung ist die Erfindung beispielsweise und schematisch dargestellt. Es zeigen:
- Fig. 1: eine erfindungsgemäße Vorrichtung mit einer in Seitenansicht dargestellten Elektrodeneinrichtung mit elektrischen Anschlüssen an einen als Blockschaltbild dargestellten Hochfrequenzgenerator,
- Fig. 2: eine Ansicht von unten der in Fig. 1 dargestellten Elektrodeneinrichtung, und
- Fig. 3: eine Darstellung gemäß Fig. 2 einer Elektrodeneinrichtung in anderer Ausführungsform.

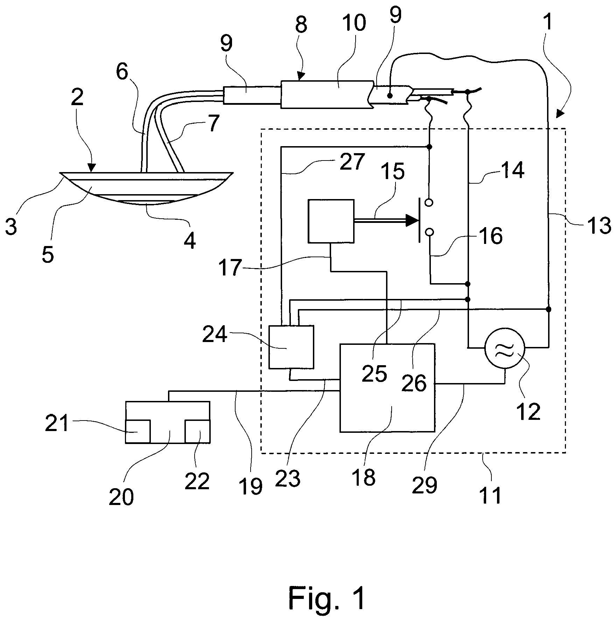

Fig. 1 zeigt eine Vorrichtung 1 zur hochfrequenzchirurgischen Behandlung einer in der Zeichnung nicht dargestellten Gewebeoberfläche. In einem häufigen Anwendungsfall soll beispielsweise in leitfähiger Flüssigkeit eine hypertrophierte Prostata durch Vaporisation abgetragen werden.

Die Vorrichtung 1 weist eine Elektrodeneinrichtung 2 auf, die eine in der Darstellung der Fig. 1 nach unten konvex vorspringende Arbeitsfläche 3 ausbildet.

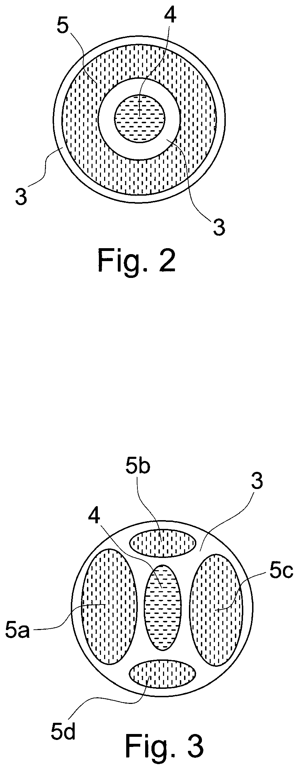

Fig. 2 zeigt die Arbeitsfläche 3 von unten. Man sieht, dass auf der Arbeitsfläche 3 zwei Elektroden flächig angeordnet ist, die im Ausführungsbeispiel der Figuren 1 und 2 aus einer mittleren ersten Elektrode 4 und einer darum ringförmig angeordneten zweiten Elektrode 5 bestehen. Unter flächiger Ausbildung der Elektroden wird hier verstanden dass die Elektroden über einen Flächenanteil der Arbeitsfläche erstreckt sind.

Die beiden Elektrode 4 und 5 sind auf der nicht näher dargestellten Oberseite der Elektrodeneinrichtung 2 mit elektrischen Leitern kontaktiert, die beide mit äußerer Isolierung versehen sind. Dabei ist ein erster Leiter 6 an die erste Elektrode 4 angeschlossen und ein zweiter Leiter 7 an die zweite Elektrode 5.

Die beiden Leiter 6, 7 verlaufen in einem langgestreckten Elektrodenträger 8. Sie sind im dargestellten Ausführungsbeispiel mit einer rohrförmigen Neutralelektrode 9 umgeben, die über die wesentliche Länge des Elektrodenträgers 8 mit einer Isolierung 10 abgedeckt ist, im distalen Endbereich, also in der Nähe der Elektrodeneinrichtung 2 jedoch als Neutralelektrode freiliegt. Wenn die dargestellte Elektrodeneinrichtung in leitfähiger Flüssigkeit liegt, kann Strom durch die Flüssigkeit zwischen den Elektroden 4 und 9 oder 5 und 9 fließen.

Die drei Elektroden 4, 5 und 9 sind, wie Fig. 1 zeigt, elektrisch an einen Hochfrequenzgenerator 11 angeschlossen. Dieser weist als Kernbestandteil eine Hochfrequenzspannungsquelle 12 auf, die zwischen zwei Polen Hochfrequenzspannung erzeugt. Der eine Pol ist über eine Leitung 13 an die Neutralelektrode 9 angeschlossen und der andere Pol über eine Leitung 14 an den ersten Leiter 6, der die erste Elektrode 4 versorgt. Der Anschluss an die Leiter des Elektrodenträgers 8 ist in Fig. 1 sehr schematisch und provisorisch dargestellt. In einem realen Anwendungsfall wäre hier eine dreipolige Steckverbindung vorgesehen.

In der bis jetzt erläuterten Konfiguration wäre die dargestellte Vorrichtung 1 grundsätzlich arbeitsfähig. Hochfrequenter Strom würde zwischen den Elektroden 4 und 9 durch Flüssigkeit oder Gewebe fließen, würde zünden und eine Plasmaschicht erzeugen und zwar nur auf der angeschlossenen ersten Elektrode 4. Da diese flächenmäßig sehr klein ist, erfolgt eine schnelle und sichere Zündung.

Anschließend kann ein Schalter 15 betätigt werden, der eine Leitung 16 schließt, womit die zweite Leitung 7 zur Leitung 14 verbunden wird. Beide Elektroden 4 und 5 sind nunmehr mit demselben Pol der Hochfrequenzspannungsquelle 12 verbunden. Plasma brennt nun auf beiden Elektroden 4 und 5, also sehr großflächig, so dass große Gewebeflächen sehr rasch bearbeitet werden können.

Fig. 3 zeigt eine Variante der Arbeitsfläche 3. Die erste Elektrode 4 ist hier ähnlich wie in Fig. 2 ausgebildet. Die zweite Elektrode 5 ist jedoch in vier Teile 5a bis 5d unterteilt, die untereinander elektrisch verbunden und an den zweiten Leiter 7 angeschlossen sind. Die Wirkung dieser Konstruktion ist entsprechend der der Fig. 2.

Die Elektrodenaufteilung der Fig. 3 ermöglicht auch andere Nutzungen. Es muss nicht die mittlere Elektrode 4 als erste Elektrode verwendet werden. Es kann z. B. die Fläche 5b als erste Elektrode verwendet werden und alle übrigen Flächenteile als zweite Elektrode. Das kann z. B. von Vorteil sein, wenn allein mit der ersten Elektrode gearbeitet werden soll, ohne die zweite Elektrode zuzuschalten. So etwas kann erforderlich sein, um sehr feine, kleinflächige Arbeiten auszuführen.

Der Schalter 15 wird über eine Leitung 17 von einer Steuereinrichtung 18 gesteuert. Diese kann eine Reihe von Parametern auswerten, um in Abhängigkeit davon den Schalter 15 zu steuern.

Zunächst ist die Steuereinrichtung 18 über eine Leitung 19 an ein Fußbedienteil 20 angeschlossen, das der Operateur bequem mit dem Fuß bedienen kann. Das Fußbedienteil 20 weist einen Hauptschalter 21 auf, der die Steuereinrichtung 18 dazu bringt, über eine Leitung 29 die Hochfrequenzspannungsquelle 12 aus- bzw. einzuschalten. Mit einem Hilfsschalter wird die Steuereinrichtung 18 dazu gebracht, über die Leitung 17 den Schalter 15 anzusteuern und die Zuschaltung der zweiten Elektrode 5 vorzunehmen, bzw. zu beenden.

Über eine Leitung 23 erhält die Steuereinrichtung Messergebnisse einer Messschaltung 24, die über Leitungen 25 und 26 die Spannung zwischen den Leitungen 13 und 14, also zwischen den beiden Polen der Hochfrequenzspannungsquelle 12 ermittelt. Bei entsprechender Auswertung kann die Messschaltung daraus z. B. feststellen, ob ein Plasma brennt. In Abhängigkeit davon kann die Steuereinrichtung 18 den Schalter 15 steuern.

Für die korrekte Steuerung ist auch die Feststellung von Gewebekontakt wichtig. Auch dafür ist die Messschaltung 24 ausgerüstet. Sie ist mit entsprechenden Leitung an die Elektrode 4, 5 und 9 angeschlossen und kann zwischen beliebigen zwei der Elekroden die Impedanz ermitteln und somit festzustellen, ob diese in Gewebekontakt stehen, oder nicht.

Die Steuereinrichtung 18 kann als Computerschaltung ausgebildet sein, die die erwähnten Effekte nach Programmen nacheinander oder kombiniert berücksichtigt und gegeneinander abwägt. Dabei müssen nicht alle der erwähnten Parameter genutzt werden. Die in Fig. 1 dargestellte Schaltung des HF-Generators 11 kann vereinfacht werden.

## Patentansprüche

1. Hochfrequenzchirurgische Vorrichtung (1) zur Behandlung einer Gewebeoberfläche,
mit einer Elektrodeneinrichtung (2), bei der auf einer zum Gewebekontakt bestimmten, konvex vorspringenden Arbeitsfläche (3) eine erste Elektrode (4) flächig ausgebildet ist, und
mit einem Hochfrequenzgenerator (11), der eine Hochfrequenzspannungsquelle (12) umfasst und an dessen einen Pol (14) die erste Elektrode (4) zum Betrieb anschliessbar ist,
wobei die hochfrequenzchirurgische Vorrichtung (1) ausgebildet und eingerichtet ist, ein Plasma in einer leitfähigen Flüssigkeit zu erzeugen,
wobei auf der Arbeitsfläche (3) eine an denselben Pol (14) anschliessbare zweite Elektrode (5) flächig ausgebildet ist,
wobei eine Steuereinrichtung (18) dazu angeschlossen und ausgebildet ist, zunächst die erste Elektrode (4) anzuschliessen, um mit der ersten Elektrode (4) ein Plasma zu erzeugen, und sodann die zweite Elektrode (5) zuzuschalten, und wobei die Steuereinrichtung (18) zur Zuschaltung nach Erkennung der Zündung des Plasmas mittels Messung der Spannung zwischen zwei Polen der Hochfrequenzspannungsquelle (12) mithilfe einer Messschaltung (24) ausgebildet ist.

2. Vorrichtung nach Anspruch 1, **dadurch gekennzeichnet, dass** die Steuereinrichtung (18) zur Zuschaltung nach Erkennung des Gewebekontaktes der Elektroden (4, 5) und zur Ermittlung des Gewebekontaktes durch Bestimmung der Impedanz zwischen zwei Elektroden (4, 5: 4, 9: 5, 9) ausgebildet ist.

3. Vorrichtung nach einem der vorhergehenden Ansprüche, **dadurch gekennzeichnet, dass** die Steuereinrichtung (18) zur Zuschaltung in Abhängigkeit von einem vom Anwender betätigbaren Schalter (22) ausgebildet ist.

4. Vorrichtung nach einem der vorhergehenden Ansprüche, **dadurch gekennzeichnet, dass** die zweite Elektrode (5) in Form getrennt angeordneter, gemeinsam zuschaltbarer Elektrodenteile (5a, 5b, 5c, 5d) ausgebildet ist.

5. Elektrodeneinrichtung (2) mit einer Steuereinrichtung (18) einer Vorrichtung (1) nach einem der vorhergehenden Ansprüche.

6. Hochfrequenzgenerator (11) mit einer Steuereinrichtung (18) einer Vorrichtung (1) nach einem der Ansprüche 1 bis 4.

## Claims

1. A high-frequency surgical device (1) for treatment of a tissue surface,
comprising an electrode unit (2), in the case of which a first electrode (4) is formed in a flat manner on a working surface (3), which is intended for tissue contact and which protrudes in a convex manner, and
comprising a high-frequency generator (11), which comprises a high-frequency voltage source (12) and to the one pole (14) of which the first electrode (4) can be connected for operation,
wherein the high-frequency surgical device (1) is formed and set up to create a plasma in a conductive liquid,
wherein a second electrode (5), which can be connected to the same pole (14), is formed in a flat manner on the working surface (3),
wherein a control unit (18) is connected and formed to initially connect the first electrode (4) to create a plasma by means of the first electrode (4), and to then additionally connect the second electrode (5), and
wherein the control unit (18) is formed to be additionally connected after detection of the ignition of the plasma by measuring the voltage between two poles of the high-frequency voltage source (12) with the help of a measurement circuit (24).

2. The device according to claim 1, **characterised in that** the control unit (18) is formed for connection after detection of the tissue contact of the electrodes (4, 5) and for detection of the tissue contact by determining the impedance between two electrodes (4, 5: 4, 9: 5, 9).

3. The device according to any one of the preceding claims, **characterised in that** the control unit (18) is formed for additional connection as a function of a switch (22), which can be actuated by the user.

4. The device according to any one of the preceding claims, **characterised in that** the second electrode (5) is formed in the form of electrode parts (5a, 5b, 5c, 5d), which are arranged separately and can be additionally connected jointly.

5. An electrode unit (2) comprising a control unit (18) of a device (1) according to any one of the preceding claims.

6. A high-frequency generator (11) comprising a control unit (18) of a device (1) according to any one of claims 1 to 4.

## Revendications

1. Dispositif chirurgical à haute fréquence (1) pour le traitement d'une surface d'un tissu,
avec un dispositif d'électrodes (2) dont une première électrode superficielle (4) est formée sur une surface de travail (3) convexe saillante destinée à entrer en contact avec le tissu, et
avec un générateur haute fréquence (11), lequel comprend une source de tension haute fréquence (12) et dont l'un des pôles (14) permet le branchement de la première électrode (4) en vue de son utilisation,
le dispositif chirurgical à haute fréquence (1) étant conçu et aménagé pour produire un plasma dans un liquide conducteur,
une seconde électrode superficielle (5) pouvant être branchée sur le même pôle (14) étant formée sur la surface de travail (3),
un dispositif de commande (18) étant branché et conçu pour tout d'abord brancher la première électrode (4) pour produire un plasma avec cette électrode (4), puis pour connecter alors la seconde électrode (5), et
le dispositif de commande (18) étant conçu pour une mise en circuit après détection de l'amorçage du plasma par la mesure, au moyen d'un circuit de mesure (24), de la tension entre deux pôles de source de tension haute fréquence (12).

2. Dispositif selon la revendication 1, **caractérisé en ce que** le dispositif de commande (18) est conçu pour une mise en circuit après détection du contact des électrodes (4, 5) avec le tissu et pour détecter le contact avec le tissu par détermination de l'impédance entre deux électrodes (4, 5: 4, 9: 5, 9).

3. Dispositif selon l'une des revendications précédentes, **caractérisé en ce que** le dispositif de commande (18) est conçu pour une mise en circuit en fonction d'un commutateur (22) pouvant être actionné par l'utilisateur.

4. Dispositif selon l'une des revendications précédentes, **caractérisé en ce que** la seconde électrode (5) est formée par des parties d'électrode (5a, 5b, 5c, 5d) agencées séparément et pouvant être mises en circuit conjointement.

5. Dispositif d'électrodes (2) avec un dispositif de commande (18) d'un dispositif (1) selon l'une des revendications précédentes.

6. Générateur haute fréquence (11) avec un dispositif de commande (18) d'un dispositif (1) selon l'une des revendications 1 à 4.
